Europäisches Patentamt

European Patent Office

Office européen des brevets

(19)

(11) Veröffentlichungsnummer: **0 201 618**
**B1**

(12) **EUROPÄISCHE PATENTSCHRIFT**

(45) Veröffentlichungstag der Patentschrifft:
21.03.90

(21) Anmeldenummer: 85106057.4

(22) Anmeldetag: 17.05.85

(51) Int. Cl.⁵: **A 61 K 37/14**

(54) Verfahren zur Polymerisation von Hämoglobin mittels verknüpfender Reagenzien.

(43) Veröffentlichungstag der Anmeldung:
20.11.86 Patentblatt 86/47

(45) Bekanntmachung des Hinweises auf die Patenterteilung:
21.03.90 Patentblatt 90/12

(84) Bennante Vertragsstaaten:
AT BE CH DE FR GB IT LI LU NL SE

(56) Entgegenhaltungen:
EP-A-0 038 172
EP-A-0 038 173
EP-A-0 078 961
DE-A-2 417 619
DE-A-2 607 706
DE-A-2 646 854
DE-A-2 714 252
DE-B-2 449 885
US-A-4 001 401

(73) Patentinhaber: Barnikol, Wolfgang, Prof. Dr.Dr.
Lanzelhohl 66
D-6500 Mainz-Bretzenheim (DE)
Patentinhaber: Burkhard, Oswald, Dr.
Schillerstrasse 9
D-6761 Kriegsfeld (DE)

(72) Erfinder: Barnikol, Wolfgang, Prof. Dr.Dr.
Lanzelhohl 66
D-6500 Mainz-Bretzenheim (DE)
Erfinder: Burkhard, Oswald, Dr.
Schillerstrasse 9
D-6761 Kriegsfeld (DE)

(74) Vertreter: Ratzel, Gerhard, Dr.
Seckenheimer Strasse 36a
D-6800 Mannheim 1 (DE)

2

**Beschreibung**

Die Erfindung betrifft ein Verfahren zur Polymerisation von Hämoglobin mittels verknüpfender Reagenzien.

Bekanntlich können sauerstofftragende Blutersatzlösungen im Katastrophenfall, also beispielsweise bei einem Operationszwischenfall mit schwer zu beherrschenden Blutungen, bei Unfällen unter Blutverlust oder für den Fall eines Infektionsrisikos (Hepatitis, AIDS = Aquiriertes Immun-Defekt-Syndrom) als Ersatz für eine momentan nicht verfügbare passende Blutkonserve infundiert werden; dies gilt insbesondere dann, wenn ein Mensch z. B. in den genannten Fällen in einen Volumen-Mangel-Schock geraten ist.

Es ist möglich, daß eine sauerstofftragende Blutersatzlösung einen Volumen-Mangel-Schock eher durchbrechen kann, als eine Blutkonserve, da die Erythrozyten bekanntlich in der Konserve versteift sind und dadurch eine verringerte Kapillardurchgängigkeit aufweisen.

An Tierversuchen ist gezeigt worden, daß mit sauerstofftragenden Blutersatzlösungen ein Volumen-Mangel-Schock wirksamer bekämpft werden kann als mit einfachen Plasmaexpandern (Übersichtliche Literaturstelle hierzu: R. Pabst, Med. Klin. 72 (1977), Seiten 1555 bis 1562).

Zur Herstellung sauerstofftragender Blutersatzmedien sind bereits verschiedene Wege beschritten worden, nämlich

1. Verwendungen von Emulsionen von Fluorkohlenwasserstoffen, in welchen der Sauerstoff sehr gut löslich ist (Übersichtliche Literaturstelle hierzu: Hirlinger et al., Anaethesist 31 (1982), Seiten 660 bis 666).
2. Die Mikroverkapselung konzentrierter Hämoglobinlösungen in Phospholipid-Vesikeln (Literaturstelle hierzu: Gaber et al., Encapsulation of Hemoglobin in Phospholipid Vesicles; Preparation and Properties of a Red Cell Surrogate in "The Red Cell, Sixth Ann Arbor Conference", G. J. Brewer (Herausgeber), Alan R. Liss, Inc., New York, 1984, Seiten 179 bis 190).
3. Herstellung geeigneter Hämoglobinlösungen, auch unter kovalenter Bindung des Hämoglobins an Dextrane.

Die DE-A-2 417 619 beschreibt beispielsweise polymerisiertes, verknüpftes Hämoglobin als Plasmaprotein-Ersatz, wobei dicarboxylidatverknüpftes Hämoglobin hergestellt wird.

Die DE-A-2 714 252 beschreibt pyridoxalphosphat-verknüpftes Hämoglobin.

Die DE-A-3 029 307 betrifft ein Blutersatzmittel, hergestellt durch kovalente Verknüpfung von Polysaccharid, beispielsweise Dextran, mit zellfreiem Hämoglobin.

Die BE-B-838 933 beschreibt ein wasserlösliches, verknüpftes, polymerisiertes Hämoglobin, das hergestellt wird durch Umsetzung freien Hämoglobins mit einem polifunktionellen verknüpfenden Agens und ausschließendem Abstoppen der Reaktion mit einem inaktivierenden Mittel. Es wird ein polymerisiertes Hämoglobin mit einem Molekulargewicht von 64 000 bis 1 000 000 Dalton erhalten.

Die US-A-4 001 401 betrifft Poly-Hämoglobin, ein verknüpftes Hämoglobin, als Blutersatz und Plasmaexpander, mit einem Molekulargewicht von 64 000 bis zu 1 000 000 Dalton. Als verknüpfende Agenzien werden Glutardialdehyd, Hexamethylendiisocyanat oder Butadiendiepoxid verwendet.

Die EP-A-38 173 betrifft künstliche rote Blutzellen, die Mikrotropfen von wässriger Hämoglobinlösung (nicht polymerisiert) darstellen, die eingekapselt sind in Membranen aus polymerisiertem Hämoglobin. Das Verfahren der Herstellung besteht darin, Mikrotropfen in einer Hämoglobinlösung in einer kontinuierlichen Ölphase zu bilden, diese Tropfen in einer wässrigen Phase zu emulgieren, wobei die wässrige Phase das vernetzende Agens enthält.

Die EP-A-38 172 betrifft ein Verfahren zur Herstellung künstlicher roter Blutzellen, bei denen Mikrotropfen von Hämoglobinlösung in Membranen aus polymerisiertem Hämoglobin eingeschlossen werden, wobei das Verfahren die Bildung von Mikrotropfen von Hämoglobin in einer kontinuierlichen Ölphase und die Vernetzung des Hämoglobins mit einem geeigneten öllöslichen Vernetzungsmittel beinhaltet. Die gewonnen künstlichen roten Blutzellen werden anschließend mittels einer wässrigen Phase aus der Ölphase extrahiert.

Die DE-A-26 07 706 betrifft ein wasserlösliches polymerisiertes vernetztes Hämoglobin, bei dem die Vernetzung unter weitgehendem Sauerstoffausschluß in einer wässrigen Lösung von Hämoglobin mit einem ebenfalls in wässriger Phase vorliegenden vernetzenden Agens stattfindet.

Die EP-A-78 961 betrifft ein Verfahren zur Herstellung eines vernetzten Hämoglobinpräparates, bei dem eine wässrige Lösung von Hämoglobin einer Sauerstoffreduzierung unterworfen wird, eine wässrige Vernetzerlösung zugeführt wird und weiterhin ein Reduktionsmittel zugeführt wird.

Die US-A-4 001 401 betrifft eine vernetzte Hämoglobinpräparation, wobei die Vernetzung einer wässrigen Phase von Hämoglobin mit einem in der wässrigen Phase gelösten vernetzenden Agens und unter Sauerstoffausschluß erfolgt.

Die DE-B-2 449 885 betrifft ein Verfahren zur Herstellung chemisch modifizierter Hämoglobinpräparate, wobei die Hämoglobinlösung in Anwesenheit anderer Proteine mit vernetzenden Dialdehyden polymerisiert, und anschließend eine fraktionierte Auftrennung durchgeführt wird.

Die DE-A-2 714 252 betrifft ein Verfahren zur Herstellung eines Hämoglobinpräparates, bei dem nach Sauerstoffreduktion die wässrige Hämoglobinlösung durch Pyridoxalphosphat und Zugabe von Natriumborhydrid sowie Dialdehyd polymerisiert und anschließend fraktioniert wird.

Die Verfahren gemäß dem Stande der Technik sind aufwendig, beinhalten oft zusätzliche Verfahren und Aufarbeitung-Schritte und liefern Hämoglobinpräparate mit unzureichender sauerstofftragender Kapazität.

Diese bekannten Verfahren können zumindest in gewisser Hinsicht nicht befriedigen.

So ist es beispielsweise beim Verfahren der US-A-4 001 401 erforderlich, Amine zur Verhinderung der Entstehung unlöslicher Produkte vor Zugabe des Verknüpfungsmittels zuzugeben. Amine können bekanntlich toxisch wirken.

Außerdem werden unzureichend polymerisierte Polyhämoglobine erhalten.

Bei Anwendung von Fluorkohlenwasserstoffen wurde gewebliche Reaktionen festgestellt (siehe oben bei Hirlinger et al.). Für die Hämoglobin-Vesikel liegen bisher keine ausreichenden invivo-Versuche vor (siehe oben bei Gaber et al.). Vermutlich stimulieren sie jedoch sehr stark das retikuloendotheliale System (siehe unten). Der routinemäßigen klinisch-praktischen Nutzung von Hämoglobinlösungen stehen bisher jeweils mehr oder weniger sechs verschiedene Probleme entgegen, nämlich:

1. Erhöhung der Sauerstoffaffinität (zu geringer Halbsättigungsdruck, $P_{50}$), so daß der in der Lunge aufgenommene Sauerstoff nur ungenügend an das Gewebe abgegeben werden kann; dies tritt ausgeprägt bei der kovalenten Bindung des Hämoglobins an Dextran auf.
2. Zu geringe Verweildauer des Hämoglobins im Gefäßsystem, Ausscheidung über die Niere.
3. Mit dem unter (2) aufgezeigten Nachteil ist ferner ein intravasaler Volumenmangel verbunden, so daß man gerade das erzeugt, was man bekämpfen will;
4. Nephrotoxizität und
5. Stimulierung des Immunsystems sowie Besetzung der phagozytierenden Kapazität des retikuloendothelialen Systems durch das infundierte Hämoglobin.
6. Überlastung des Organismus mit Lipoiden (Emulgatoren), tritt bei Herstellung von Hämoglobinvesikeln.

Es wurde gezeigt, daß zur Einstellung eines hohen gemischt venösen $O_2$-Partialdrucks nicht so sehr ein hoher Halbsättigungs-Druck ($P_{50}$) erforderlich ist (ein $P_{50}$ von 15 mmHg ist nämlich ausreichend), als vielmehr eine hohe Sauerstoffbindungskapazität im Plasma, d.h. eine hohe Massenkonzentration des Hämoglobins (Literaturstelle hierzu : Moss et al., "Hemoglobin Solution -From Tetramer to Polymer", in "The Red Cell: Sixth Ann Arbor Concference", G.J. Brewer (Herausgeber), Seite 191 bis 220, Alan R. Liss, Inc. New York, 1984). Um das eben genannte Erfordernis zu befriedigen und gleichzeitig eine geringe onkotische Wirksamkeit zu erreichen, muß das Hämoglobin zu Molekülen mit möglichst hohem Polymerisationsgrad polymerisiert werden. Bisher wurden mittlere Polymerisationsgrade von 10, d.h. mittlere Molekulargewichte von etwa 700 000 Dalton erreicht.

Der vorliegenden Erfindung liegt nun die Aufgabe zugrunde, natürliche Hämoglobine bis zu einem möglichst hohen Polymerisationsgrad, mindestens bis zu einem mittleren Molekulargewichte von $3 \cdot 10^6$ Dalton zu polymerisieren, damit in den anzuwendenden Konzentrationen ihr kolloidosmotischer Druck gegen den des Plasmas ($\sim 34$ mbar) zu vernachlässigen ist. Diese erfindungsgemäß hergestellten sehr hoch polymeren löslichen Hämoglobine dienen in erster Linie zur Produktion sauerstofftragender Blutersatzlösungen.

Die vorgenannte Aufgabe wird beim erfindungsgemäßen Verfahren zur Polymerisation von Hämoglobin mittels verknüpfender Reagenzien dadurch gelöst, daß man das Hämoglobin in hochkonzentrierten Lösungen bei Konzentrationen größer als 20 g/dl durch Einwirkung polyfunktioneller Agenzien polymerisiert, wobei die polyfunktionellen Agenzien aus einer anderen Phase zugeführt werden. d.h. eine inhomogene Polymerisation durchgeführt wird.

Bevorzugte Ausführungsformen sind dadurch gekennzeichnet, daß man bei Konzentrationen größer als 30 g/dl polymerisiert, daß man Polymerisate mit einem Molekulargewicht von 60 000 bis mehr als $6 \cdot 10^6$ Dalton herstellt und daß die Phase, aus der die polifunktionellen Agenzien kommen, eine feindisperse ist.

Noch weitere besondere Ausführungsformen sind dadurch gekennzeichnet, daß die Polymerisation an intakten Erythrozyten durchgeführt wird,

- daß die Polymerisation an osmotisch geschrumpften Erythrozyten durchgeführt wird,
- daß die Polymerisation bei einer Temperatur zwischen 0°C und 40°C durchgeführt wird,
- daß man Glutardialdehyd als polifunktionelles Agens verwendet und
- daß der Hämatokrit zwischen 0,1 und 99,9 Vol.-% liegt.

Es ist auch möglich, direkt und ohne weitere Präparation das Hämoglobin im intakten Erythrozyten selbst zu polymerisieren. Es liegt hier in einer Konzentration von etwa 35 g/dl vor.

Um die Konzentration des Hämoglobins im Erythrozyten vor Zugabe des verknüpfenden Agens noch weiter zu steigern, und damit eine weitere Erhöhung des Polymerisationsgrades zu erzielen, können die Zellen in hypertonen Natriumchloridlösungen geschrumpft werden. Allerdings kann daß verknüpfende Agens (z. B. Glutardialdehyd) bei diesen hohen Konzentrationen nicht direkt zugesetzt werden. Es entstände nämlich, insbesondere direkt an der Zugabestelle, momentan ein unlösliches Produkt. Das verknüpfende Agens wird vielmehr indirekt zugegeben, und zwar aus einer fein verteilten nichtwässrigen Phase. Das verknüpfende Agens wird in der nicht wässrigen Phase vorher gelöst. Die Reaktion wird zwischen 0 und 40°C ausgeführt. Der Hämatokrit liegt zwischen 0,1 und 99,9 Vol.-% unter Anpassung des zugesetzten Volumens an mit Glutardialdehyd gesättigtem Chloroform. Die Reaktion dauert je nach der gewählten Temperatur zwischen einigen Minuten und mehreren Stunden.

Das erzielte hohe Molekulargewicht des löslichen Hämoglobins erleichtert wesentlich eine nachfolgende (insbesondere chromatographische) Reinigung des Polymerisats von anderen Substanzen. Die Möglichkeit, die Polymerisation direkt am intakten Erythrozyten durchzuführen, macht eine vorhergehende aufwendige biotechnologische Präparation der hochkonzentrierten Hämoglobinlösung überflüssig, womit man auch eine unerwünschte Bildung von inaktivem Met-Hämoglobin vermeidet.

Das Wesen vorliegender Erfindung wird nun anhand der folgenden Ausführungsbeispiele, die bevorzugte Ausführungsformen zeigen, weiterhin erläutert:

**Beispiel 1**

**Polymerisation einer hochkonzentrierten Hämoglobinlösung**

Blut wird von einem gesunden Menschen aus einer Vene entnommen und mit Heparin ungerinnbar gemacht. Die Erythrozyten werden zur Entfernung der Plasmas mit isotonischer Natriumchloridlösung (0,9 g/dl) zwei- bis dreimal unter Zentrifugieren gewaschen. Zum Schluß erfolgt eine scharfe Zentrifugation (15 Minuten bei 15 000 g), damit die Erythrozyten dicht gepackt sind.

Die gepackten Zellen werden durch Eintauchen des Röhrchens nach Absaugen der überstehenden Lösung in flüssige Luft durch Kälteschock zerstört (Kryolyse). Das Zellhomogenat wird mit "extrazellulärer" Elektrolytlösung (diese enthält in mmol/l: 145 NaCl; 4 KCl; 1,45 $CaCl_2$) etwa 1 : 20 verdünnt, scharf zentrifugiert und die überstehende klare Hämoglobin-Lösung zur Entfernung von Rest-Stroma durch 0,22 µm Filter (Millipore®, Neu-Isenburg, Bundesrepublik Deutschland) filtriert. Die Lösung wird darauf mittels Ultrafiltration mit dem TCF 10® und /oder TCF 2® (Amicon, Witten, Bundesrepublik Deutschland) an PM 10- oder PM 30-Membranen auf etwa 35 g/dl konzentriert.

1 ml der hochkonzentrierten Hämoglobinlösung werden in einem 25 ml-Becherglas mit 120 µl einer Glutardialdehydlösung in Chloroform versetzt und mit einem Magnetrührer bei 200 Umdrehungen pro Minute und 22°C gerührt. Die Glutardialdehydlösung stellt man kurz zuvor her, indem man 25 %-ige wässrige Aldehydlösung im Verhältnis 1 : 1 mit Chloroform zusammengibt und kräftig schüttelt. Nach etwa 1,5 Stunden unterbricht man die Reaktion durch Verdünnen und zentrifugiert scharf. Man erhält in der überstehenden Lösung lösliches Hämoglobin mit einer Ausbeute von etwa 75 % mit einem Met-Hämoglobingehalt von 7 %. Das lösliche Hämoglobin ist zu 30 % monomer (64 000 Dalton) und zu 70 % polymer (mittleres Molekulargewicht 3 · $10^6$ Dalton), so daß man eine Gesamtausbeute an polymerem Hämoglobin von etwa 50 % erreicht. Die Molekulargewichtsbestimmung erfolgt mit Hilfe der Dünnschichtgelchromatographie an geeigneten Gelen (Sephadex oder Sephacryl : Deutsche Pharmacia, Freiburg, Bundesrepublik Deutschland). Als Eichsubstanzen wurden monomeres Hämoglobin (64 000 Dalton), Blaues Dextran ( 2 · $10^6$ Dalton) und Erythrocruorin vom Regenwurm (3 · $10^6$ Dalton) verwendet.

**Beispiel 2**

**Polymerisation in intakten Erythrozyten**

Die Zellen werden gewonnen und gereinigt wie im Beispiel 1. Sie werden dann im "extrazellulären" Milieu (siehe oben) mit einem Hämatokrit von 50 % aufgeschwemmt. 3 ml der Suspension werden in ein 25 ml-Becherglas gegeben, die Polymerisation wird wie im Beispiel 1 ausgeführt. Während der Reaktion tritt Hämolyse ein. Nach etwa 1,5 Stunden wird die Reaktion durch Verdünnen mit "extrazellulärem" Milieu unterbrochen. Es folgt eine Zentrifugation. Die überstehende klare Hämoglobinlösung hat die gleichen Eigenschaften wie im Beispiel 1, auch die Ausbeute ist gleich groß.

Die Polymerisate beider Beispiele haben auch gleiche sauerstoffbindende Eigenschaften. Der Halbsättigungsdruck einer 1,5 %-igen Lösung beträgt bei pH = 7,40 und bei einem $CO_2$-Partialdruck von 40 mmHg, gemessen gemäß Barnikol et al. (Respiration 36 (1978), Seiten 86 bis 95), 13 mmHg. Der mittlere Hillsche Index (n) ist gleich 2,0.

**Patentansprüche**

1. Verfahren zur Polymerisation von Hämoglobin mittels verknüpfender Reagenzien, *dadurch gekennzeichnet*, daß man das Hämoglobin in hochkonzentrierten Lösungen bei Konzentrationen größer als 20 g/dl durch Einwirkung polyfunktionellen Agenzien polymerisiert, wobei die polyfunktionellen Agenzien von einer anderen Phase aus zugeführt werden, d. h. eine inhomogene Polymerisation durchgeführt wird.

2. Verfahren nach Anspruch 1, *dadurch gekennzeichnet*, daß man bei Konzentrationen größer als 30 g/dl polymerisiert.

3. Verfahren nach Anspruch 1 bis 2, *dadurch gekennzeichnet*, daß man Polymerisate mit einem Molekulargewicht von 60 000 bis mehr als 6 · $10^6$ Dalton herstellt.

4. Verfahren nach Anspruch 1 bis 3, *dadurch gekennzeichnet*, daß die Phase, aus der die polifunktionellen Agenzien kommen, eine feindisperse ist.

5. Verfahren nach Anspruch 1 bis 4, *dadurch ge-*

kennzeichnet, daß die Polymerisation an intakten Erythrozyten durchgeführt wird.

6. Verfahren nach Anspruch 1 bis 5, *dadurch gekennzeichnet*, daß die Polymerisation an osmotisch geschrumpften Erythrozyten durchgeführt wird.

7. Verfahren nach Anspruch 1 bis 6, *dadurch gekennzeichnet*, daß die Polymerisation bei einer Temperatur zwischen 0°C und 40°C durchgeführt wird.

8. Verfahren nach Anspruch 1 bis 7, *dadurch gekennzeichnet*, daß man Glutardialdehyd als polifunktionelles Agens verwendet.

9. Verfahren nach Anspruch 1 bis 8, *dadurch gekennzeichnet*, daß der Hämatokrit zwischen 0,1 und 99,9 Vol.-% liegt.

## Claims

1. Process for the polymerization of hemoglobin by means of cross-linking agents, *characterized in* that the hemoglobin is polymerized in highly concentrated solutions at concentrations higher than 20 g/dl by means of polyfunctional agents, whereby the polyfunctional agents are added from another phase, that is an inhomogeneous polymerization is carried out.

2. Process according to claim 1, *characterized in* that polymerization is carried out in concentrations higher than 30 g/dl.

3. Process according to claim 1 - 2, *characterized in* that polymerisation with a molecular weight of 60 000 to more than $6 \cdot 10^6$ Dalton are produced.

4. Process according to claim 1 - 3, *characterized in* that the phase out of which the polyfunctional agents come is a fine-disperse one.

5. Process according to claim 1 - 4, *characterized in* that the polymerization is carried out with intact erythrocytes.

6. Process according to claim 1 - 5, *characterized in* that the polymerization is carried out with osmotically shrunk erythrocytes.

7. Process according to claim 1 - 6, *characterized in* that the polymerization is carried out at a temperature between 0°C and 40°C.

8. Process according to claim 1 - 7, *characterized in* that as polyfunctional agent glutardialdehyde is utilized.

9. Process according to claim 1 - 8, *characterized in* that the hematocrit is between 0,1 and 99, 9 Vol.-%.

## Revendications

1. Procedé pour la polymérisation de la hémoglobine par des agents enchainants, *characterisé par* qu'on polymérise la hémoglobine en solutions très concentrées à des concentrations plus grands que 20g/dl par action des agents polyfonctionels, en ajoutant les agents polyfonctionels d'une autre phase, c'est-à-dire qu'on réalise une polymérisation inhomogène.

2. Procedé selon revendication 1, *characterisé par* qu'on polymérise aux concentrations plus grands que 30g/dl.

3. Procedé selon revendications 1 jusqu'à 2, *characterisé par* qu'on produit des polymères avec un poids moléculaire de 60 000 jusqu'à plus de $6 \cdot 10^6$ Dalton.

4. Procedé selon revendications 1 jusqu'à 3, *characterisé par* que la phase, de laquelle les agents polyfonctionels viennent, est une phase fin dispersée.

5. Procedé selon revendications 1 jusqu'à 4, *characterisé par* que la polymérisation est effectuée avec des erythrocytes intacts.

6. Procedé selon revendications 1 jusqu'à 5, *characterisé par* que la polymérisation est effectuée avec des erythrocytes rétrécissés osmotiquements.

7. Procedé selon revendications 1 jusqu'à 6, *characterisé par* que la polymérisation est effectuée à une température entre 0°C et 40°C.

8. Procedé selon revendications 1 jusqu'à 7, *characterisé par* qu'on utilise aldéhyde glutarique comme agent polyfonctionel.

9. Procedé selon revendications 1 jusqu'a 8, *characterisé par* que l'hématocrit est situé entre 0,1 et 99,9 pourcent volumétrique.